# EUROPEAN PATENT SPECIFICATION

(11) **EP 1 919 777 B1**
(45) Date of publication and mention of the grant of the patent: **06.03.2019**
(21) Application number: 06758056.3
(22) Date of filing: 13.07.2006
(51) Int. Cl.: B65B 55/10, B65B 39/04

(54) **A METHOD AND AN APPARATUS FOR STERILISING PACKAGES**
VERFAHREN UND VORRICHTUNG ZUM STERILISIEREN VON VERPACKUNGEN
PROCEDE ET APPAREIL DE STERILISATION D EMBALLAGES

(30) Priority: 23.08.2005 SE 0501872
(43) Date of publication of application: 14.05.2008
(73) Proprietor: Tetra Laval Holdings & Finance S.A., 1009 Pully (CH)
(72) Inventor: LINDBLAD, Ulf, S-224 56 Lund (SE); OLSSON, Jenny, Karolina, S-237 33 Bjärred (SE); SUNDBERG, Anders, S-247 91 Södra Sandby (SE)
(74) Representative: Tetra Pak - Patent Attorneys SE
(86) International application number: PCT/SE2006/000874
(87) International publication number: WO 2007/024172

(56) References cited:
- DE-A1- 3 339 930
- FR-A1- 2 774 912
- JP-A- 2000 202 388
- JP-A- 2000 202 388
- JP-A- 2002 102 813
- US-A- 2 492 873
- US-A- 2 771 645
- US-A- 5 141 035

## Description

### TECHNICAL FIELD

The present invention relates to a method and an apparatus for sterilising at least partly formed packages which are ready-to-fill packages, in a filling machine. The term sterile is taken to signify in the following disclosure that the package, after sterilisation, attains a level of sterilisation which is designated commercially sterile.

More precisely, the present invention relates to a method, prior to filling of such packages, of treating and sterilising them in a filling machine before a subsequent aseptic filling. The packages have an open and a closed end. A first context in which the method according to the present invention may be implemented is in connection with the introductory supply, before filling of such packages, of hot air from the open end of the packages in order to heat them up with a view, in a later sterilisation stage, to preventing sterilisation gas from condensing on the walls of the packages. Another manner of implementing the method is to supply sterile tempered air once the package has been gassed with sterilisation gas. The purpose here is to ventilate off the sterilisation gas. More specifically, the present invention relates to a method of supplying and removing an optional gas, hence also sterilisation gas to and from the open end of the package.

The present invention further relates to an apparatus which is included in a larger context for realising a gas sterilisation of packages in said filling machine where the larger context includes, on the one hand, a heating zone, and on the other hand a sterilisation zone or a combination thereof and further a ventilation zone. The sterilisation agent is intended to remain in gas form throughout the entire sterilisation stage and is intended to the greatest possible degree to be reused. For a more detailed description of one type of an apparatus and a method for producing and sterilising a package which is referable to this group, reference is made to published international application WO2004/054883.

### BACKGROUND ART

In filling machines of said type, use has previously been made of a method of approach which entails that, during the gas sterilisation stage, a sterilisation gas is supplied centrally in conjunction with the open end of each package. For reasons of process engineering, the sterilisation gas flow has been allowed to remain constant, regardless of the necessary movements in the filling machine. This is linked to the situation that the relevant technology is applicable in connection with filling machines of different types, such as also those of the tube filling- or carousel filling type. It has proved that, in certain cases, there may be a minor risk that the sterilisation gas which, in a previously known manner, is supplied to packages of this type in some cases does not reach the small areas and pockets of the package which are located least readily accessible in relation to the sterilisation agent supplying device. This applies particularly to such packages which display a relatively large ratio between their length and their main axis cross section. It has long been assumed that it is possible to reach these small areas and pockets of the packages located least readily accessible by maintaining a high constant gas mass flow in the supplied sterilisation gas. However, it has now been established that, above all in certain cases, there is a risk that the preselected level of sterilisation will not be achieved simply by this measure. DE-A-3339930 relates to a gas supplying apparatus in a filling machine of the generic kind, wherein the gas is supplied by means of a ring-shaped nozzle such that the gas flow is directed vertically downwards along the inner side wall of a cup-shaped package.

### BRIEF SUMMARY OF THE INVENTION

One object of the present invention is to disclose a method, with the best conceivable overriding control prior to filling of a ready-to-fill package under aseptic conditions, to carry out a gas sterilisation process of the inside of said package to the preselected level of sterilisation, with a considerably lower gas mass flow of the supplied gases or gas mixtures as compared with the prior art.

More specifically, one object of the present invention is to disclose a method which, in particular in the specifically disclosed case, obviates the risk that the sterilisation will be incomplete, at the same time as the mass flow of the supplied gases is greatly reduced compared with the prior art.

Yet a further object of the present invention is to realise a method which makes for a considerably higher level of reusing of the supplied sterilisation gas.

Yet a further object of the present invention is to disclose an apparatus for, on the one hand, realising a reduction of the risk of re-infection or contamination with a view to better being able to guarantee the shelf-life of the product which is subsequently filled into the package, and, on the other hand, for realising a reduction of the gas mass flow in connection with the sterilisation and thereby improve the economy of the process, whereby considerably improved possibilities will occur for reusing, in particular concerning the supplied sterilisation gas. As a result, the unintentional spreading of the sterilisation gas in the aseptic chamber can be restricted to an even higher degree than before. Said chamber consists, on the one hand, of a unit for the supply of hot air, and, on the other hand, of a unit for the supply and removal of sterilisation agent, and also a unit for ventilation of packages of sterilisation gas before they are filled.

According to the present invention, there is provided in an apparatus in a filling machine a method of supplying a gas air gas mixture to the inside of partly formed packages whjch are ready-to-fill, before subsequent filling and sealing of the packages. The gas or gas mixture supplied at any given moment is supplied as a flow which is radially inwardly defined in relation to the inner wall of the package, such that the flow is angled with respect to the geometric major longitudinal axis (H) of the package which intersects its opening so that the flow, since it is defined by the inner wall of the package, is controlled for the formation of a helical flow vortex. The thus created flow vortex forms, as a consequence that it must, by some means, find a way out again, a central return flow thanks to a lower gas pressure in this central region. Hereby, the gas velocity may, regardless of the gas type which is employed, be reduced. One advantage inherent herein is that it is thereby possible to eliminate positive flow vortices in the treatment chamber. Furthermore, there will be provided according to the invention an apparatus in a filling machine for supplying gas or gas mixtures to the inside of packages before a subsequent filling and sealing of the packages., The apparatus includes a nozzle for the flow of gas or gas mixture which includes at least one channel for the gas or gas mixture which is angled in relation to at least one plane containing that one of the geometric major axes of the package which intersects the opening of the package at a right angle such that the flow therefrom, when it impinges on the inner wall of the package, develops a both radially inwardly defined flow while forming a helical flow vortex. In order to attain the best possible flow distribution when gas is supplied, it has proved in trials hitherto that the supply means should encompass some ten-odd apertures, where each one of them moreover advantageously should be angled in such a manner that it makes an angle of less than 8° to each one of the two planes of symmetry to the package which have the axis of symmetry intersecting the opening under a right angle as a common line. This implies more precisely that each aperture is directed in a first direction slightly peripherally and in a second direction slightly towards the centre of the package.

There will hereby be attained not only the advantage that a helical flow which reaches all pockets and nooks everywhere inside the package occurs, including the lower region, but also that the supplied gas, in a highly elegant and controlled manner, will depart from the package in a flow which is counter-directed to the helical flow and which takes place in or close to the centre of the package. This implies at the same time that a reuse of the supplied gases may simply be put into effect, for example in that an outlet may be provided in association with the centre of the gas supply apparatus according to the invention.

### BRIEF DESCRIPTION OF THE ACCOMPANYING DRAWINGS

The present invention will now be described in greater detail hereinbelow, with reference to one embodiment shown on the accompanying Drawings. In the accompanying Drawings:
Fig. 1 is a cross sectional view of an apparatus according to the invention as an explicit illustration of the function of the invention;
Fig. 2 is an isometric view of an actual apparatus according to the present invention;
Fig. 3 is an isometric exploded view of the same apparatus; and
Fig. 4 is a cross section through the centre of the apparatus in question with a package intended for sterilisation located beneath.

### DETAILED DESCRIPTION OF THE INVENTION

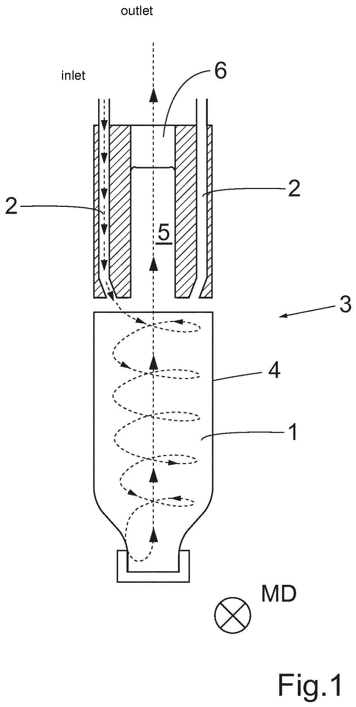

The cross sectional view illustrated in Fig. 1 shows the fundamental function of the invention. A package 1 in a sequence of identical packages and with, in this case, an open bottom runs along a belt for the progressive indexing of, for example, four packages 1 at a time in the machine direction MD. Those stages through which the package at least thereby substantially passes are one for heating the package to about 70°C, one for sterilising the package with gaseous hydrogen peroxide or other sterilisation gas, and one for ventilation thereof with sterile air. In this context, it should be observed that these stages need not be wholly single action stages but, if desired, may be "phased" in the sense that, for example, the heating stage may progressively be mixed with the sterilisation stage in an increasing degree in the machine direction so that the number of sterilisation gas apertures increases progressively. With such a modification of the method of approach, a favourable effect in the form of a temporarily longer and thereby more efficient sterilisation will be achieved.

Once these stages have been passed through, filling takes place followed by sealing of each respective package 1. Regardless of whether it is hot air, sterilisation gas or sterile ventilation air which is supplied in the channels 2 illustrated in Fig. 1 in an apparatus 3 according to the invention, it may be ascertained that the flow which results in that the channels 2 are, in their lower ends, angled in such a manner that they make an angle of less than or equal to 8° to both of the planes of symmetry which display the symmetry axis H which intersects the opening of the package at a right angle as a common line, hence angled in a first direction somewhat peripherally and in a second direction slightly towards the centre of the package imparts the best conceivable vortex helical form to the gas flow angled down in the package on its way down. In order moreover when gas is supplied to achieve the best possible flow distribution all the way down in the "bottom" of the package 1, the number of channels should, as experiments have also demonstrated, be adapted to the volume of the packages or their circumference in association with the open end of the packages; the larger the volume/circumference the greater the number of channels. In the embodiment illustrated here, the apparatus includes ten channels 2 uniformly distributed along the upper region of the described apparatus 3. It is once again worthy of pointing out that the experiments which have been carried out with water as a medium have demonstrated that the medium which is fed via the channels 2 with this configuration will flow out of the package as a substantially central flow upwards in the package 1 in order, at its upper region 4, to be able to flow into a central return channel 5. As is intimated by broken lines in the upper part of the figure, the channel 5 may discharge straight up at reference numeral 6. The desired effect will thereby be attained depending upon the quantity of supplied gas, either as improved heating, improved sterilisation or improved ventilation.

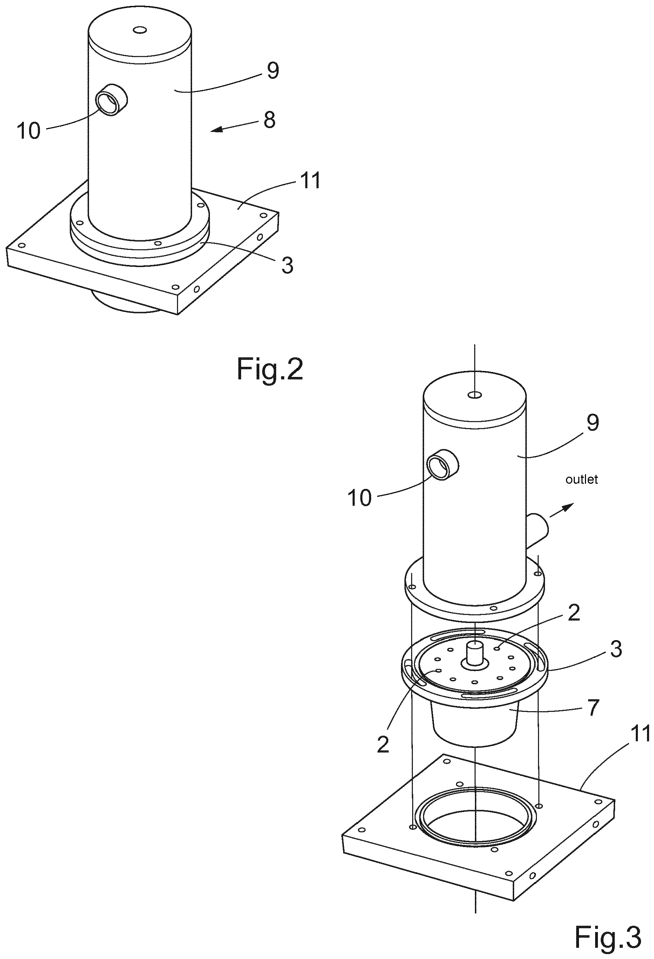

Fig. 2 shows a gassing assembly 8 comprising an apparatus 3 according to the invention in an integrated composite state with the assembly 8. The assembly 8 has an inlet chamber 9 with a central gas inlet connection 10 and a plate 11 for fixing of the assembly to a filling machine (not shown). In association with Fig. 2, Fig. 3 is a perspective exploded view of the assembly 8. Thus, the apparatus 3 is also shown here in perspective, for which reason its various component parts, above all the channels 2 are clearly apparent. The fact that the channels 2 are ten in number is, as was mentioned above, a coincidence, since the number depends upon the circumference.

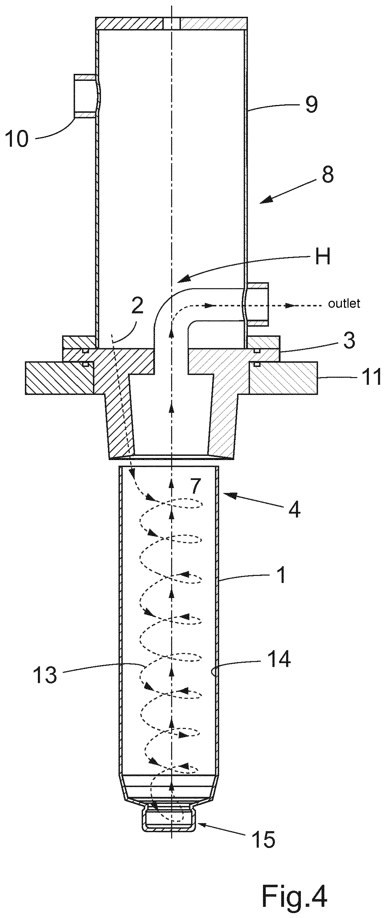

Finally, Fig. 4 shows the assembly 8 as a central cross section in a position corresponding to that which the assembly 8 will have when it is run in production. With a view to making a comparison with Fig. 1 possible, this figure shows an imaginary flow pattern corresponding to that which is illustrated in the fundamental outline drawing in Fig. 1.

A brief description will be given below of the fundamental operation of the apparatus. Supply with gas of the desired type takes place continuously at the central inflow connection 10. In that the supplied gas first fills the inflow chamber 9, the flow which is fed to the package 1 via the channels 2 will be able to maintain a uniform and constant pressure. The channels 2 are obliquely inclined in the above described manner (less than 8° in relation to two mutually right angled planes of symmetry) thereby gives rise to a helical gas flow 13 along the inner periphery 14 of the package. When the gas flow reaches the bottom 15 of the package 1, the flow will, as a consequence of the lower gas pressure in the centre of the package, strive to leave the package in this section. Thus, the return flow also takes place out of the package of the supplied gas in a controlled manner. When the return flow reaches the bottom opening of the package 1, this is taken care of in the return channel 5, in whose upper region this is deflected approx. 180° in order to be led out via the outer periphery of the package. The present invention makes for a considerable reduction of the mass flow, whereby this flow no longer constitutes a potential risk for turbulent currents occurring in the interface region beside and beneath the package.

## Claims

1. In an apparatus (3) in a filling machine, a method of supplying a gas or gas mixture to the inside of partly formed packages (1) which are ready-to-fill, before a subsequent filling and sealing of the packages,
**characterised in that** the gas or gas mixture supplied at any given moment is supplied as a flow which is radially inwardly defined in relation to the inner wall of the package (1), the flow being angled with respect to geometric major longitudinal axis (H) of the package (1) which intersects its opening so that the flow, since it is defined by the inner wall of the package is controlled for the formation of a helical flow vortex.

2. The method as claimed in Claim 1 , **characterised in that** the interior of the packages (1) is supplied, progressively via the open end of each package, on the one hand with a sterile hot air flow, on the other hand with a gaseous sterilisation agent flow, and also a sterile air flow.

3. The method as claimed in Claim 1 , **characterised in that** a gaseous sterilisation agent is progressively admixed to the initially supplied hot air gas flow in the machine direction (MD).

4. The method as claimed in Claim 1 , **characterised in that** the radial defining of the flow inwardly in the package (1) is restricted by a return flow in the central region of each package.

5. The method as claimed in any of the preceding Claims, **characterised in that** the flow is supplied by means of two or more channels (2) per open package end.

6. The method as claimed in Claim 4, **characterised in that** the channels (2) are angled so that the flow directions therefrom fall outside both of the planes of symmetry which have the axis of symmetry (H) which at right angles intersects the opening of the package as a common line, i.e. are angled in a first direction somewhat peripherally and are angled in a second direction slightly towards the centre of the package (1).

7. The method as claimed in Claim 6, **characterised in that** the channels (2) are angled less than or equal to 8[deg.] in each of the first and second directions, respectively.

8. The method as claimed in any of the preceding Claims, **characterised in that** the flow from the nozzles is maintained continuously over time.

9. The method as claimed in any of the preceding Claims, **characterised in that** the supplied gas flow, at least when it exclusively consists of sterilisation agent, is recycled for reuse **in that** an outlet (6) is provided centrally at the mouth of each respective package (1).

10. An apparatus in a filling machine for supplying gas or gas mixtures to the inside of packages (1) before a subsequent filling and sealing thereof (1), **characterised in that** it includes a nozzle (3) for flow of gas or gas mixture which includes at least one channel (2) for gas or gas mixture which is angled in relation to at least one plane containing that one of the geometric major axes (H) of the package which intersects the opening of the package at a right angle such that the flow therefrom, when it impinges on the inner wall of the package, develops a both radially inwardly defined flow while forming a helical flow vortex.

11. The apparatus as claimed in Claim 10, **characterised in that** the channels (2) are angled so that their flow direction falls outside planes which are parallel with any of the planes of symmetry which have the axis of symmetry (H) which intersects the opening of the package (1) at right angles as a common line, and are directed in a first direction somewhat peripherally and in a second direction slightly towards the centre of the package (1).

12. The apparatus as claimed in Claim 11 , **characterised in that** the angling of the channels (2) is less than or equal to 8[deg.] in each respective direction.

13. The apparatus as claimed in any of Claims 10 to 12, **characterised in that** it includes a gas recycling equipment in the form of an outlet (6) which is centrally placed in association with the mouth of each respective package (1).

## Patentansprüche

1. Verfahren in einer Vorrichtung (3) in einer Füllmaschine zum Zuführen eines Gases oder Gasgemischs in das Innere teilweise geformter Verpackungen (1), die zum Füllen bereit sind, vor einem nachfolgenden Füllen und Versiegeln der Verpackungen,
**dadurch gekennzeichnet, dass** das Gas oder Gasgemisch, das in einem bestimmten Moment zugeführt wird, als ein Strom zugeführt wird, der in Bezug auf die innere Wand der Verpackung (1) radial nach innen definiert ist, wobei der Strom in Bezug auf die geometrische Hauptlängsachse (H) der Verpackung (1), deren Öffnung sie schneidet, abgewinkelt ist, sodass der Strom, da er von der inneren Wand der Verpackung definiert wird, zur Bildung eines schraubenförmigen Strömungswirbels gesteuert wird.

2. Verfahren nach Anspruch 1, **dadurch gekennzeichnet, dass** das Innere der Verpackungen (1) nach und nach über das offene Ende jeder Verpackung zugeführt wird, auf der einen Seite mit einem sterilen Heißluftstrom, auf der anderen Seite mit einem gasförmigen Sterilisationsmittelstrom und auch einem sterilen Luftstrom.

3. Verfahren nach Anspruch 1, **dadurch gekennzeichnet, dass** ein gasförmiges Sterilisationsmittel dem anfänglich zugeführten Heißluftgasstrom in der Maschinenrichtung (Machine Direction, MD) nach und nach zugemischt wird.

4. Verfahren nach Anspruch 1, **dadurch gekennzeichnet, dass** das radiale Definieren des Stroms ins Innere der Verpackung (1) durch einen Rückstrom in der mittleren Region jeder Verpackung beschränkt wird.

5. Verfahren nach einem der vorangehenden Ansprüche, **dadurch gekennzeichnet, dass** der Strom durch zwei oder mehr Kanäle (2) je offenem Verpackungsende zugeführt wird.

6. Verfahren nach Anspruch 4, **dadurch gekennzeichnet, dass** die Kanäle (2) abgewinkelt sind, sodass die Strömungsrichtungen von dort außerhalb der beiden Symmetrieebenen liegen, welche die Symmetrieachse (H) aufweisen, die in rechten Winkeln die Öffnung der Verpackung als eine gemeinsame Linie schneidet, d. h., in einer ersten Richtung leicht peripher abgewinkelt sind und in einer zweiten Richtung leicht in Richtung der Mitte der Verpackung (1) abgewinkelt sind.

7. Verfahren nach Anspruch 6, **dadurch gekennzeichnet, dass** die Kanäle (2) in der ersten bzw. zweiten Richtung um weniger oder gleich 8 Grad abgewinkelt sind.

8. Verfahren nach einem der vorangehenden Ansprüche, **dadurch gekennzeichnet, dass** der Strom von den Düsen im Laufe der Zeit kontinuierlich aufrechterhalten wird.

9. Verfahren nach einem der vorangehenden Ansprüche, **dadurch gekennzeichnet, dass** der zugeführte Gasstrom mindestens dann, wenn er ausschließlich aus Sterilisationsmittel besteht, zur Wiederverwendung zurückgeführt wird, und dadurch, dass an dem Mund jeder entsprechenden Verpackung (1) ein Auslass (6) mittig bereitgestellt ist.

10. Vorrichtung in einer Füllmaschine zum Zuführen von Gas oder Gasgemischen in das Innere von Verpackungen (1) vor einem nachfolgenden Füllen oder Versiegeln derselben (1), **dadurch gekennzeichnet, dass** sie eine Düse (3) für einen Strom von Gas oder Gasgemisch beinhaltet, welche mindestens einen Kanal (2) für Gas oder Gasgemisch beinhaltet, der in Bezug auf mindestens eine Ebene, welche die eine der geometrischen Hauptachsen (H) der Verpackung umfasst, welche die Öffnung der Verpackung in einem rechten Winkel schneidet, abgewinkelt ist, sodass der Strom von dort, wenn er auf die innere Wand der Verpackung auftrifft, einen radial nach innen definierten Strom entwickelt, während er einen schraubenförmigen Strömungswirbel bildet.

11. Vorrichtung nach Anspruch 10, **dadurch gekennzeichnet, dass** die Kanäle (2) abgewinkelt sind, sodass deren Strömungsrichtung außerhalb von Ebenen liegt, die parallel mit jeder der Symmetrieebenen sind, welche die Symmetrieachse (H) aufweisen, die die Öffnung der Verpackung (1) in rechten Winkeln als eine gemeinsame Linie schneidet, und in einer ersten Richtung leicht peripher und in einer zweiten Richtung leicht in Richtung der Mitte der Verpackung (1) ausgerichtet sind.

12. Vorrichtung nach Anspruch 11, **dadurch gekennzeichnet, dass** die Abwinklung der Kanäle (2) in jeder entsprechenden Richtung kleiner oder gleich 8 Grad beträgt.

13. Vorrichtung nach einem der Ansprüche 10 bis 12, **dadurch gekennzeichnet, dass** sie eine Gasrückführausrüstung in der Form eines Auslasses (6) beinhaltet, der in Verbindung mit dem Mund jeder entsprechenden Verpackung (1) mittig platziert ist.

## Revendications

1. Dans un appareil (3) dans une machine de remplissage, procédé de fourniture d'un gaz ou d'un mélange de gaz à l'intérieur de paquets partiellement formés (1) qui sont prêts à être remplis, avant un remplissage ultérieur et une fermeture étanche des paquets,
**caractérisé en ce que** le gaz ou le mélange de gaz fourni à n'importe quel moment donné est fourni sous la forme d'un flux qui est défini radialement vers l'intérieur par rapport à la paroi interne du paquet (1), le flux étant incliné par rapport à un axe géométrique longitudinal principal (H) des paquets (1) qui coupe son ouverture de telle sorte que le flux, puisqu'il est défini par la paroi interne du paquet, soit régulé pour la formation d'un vortex de flux hélicoïdal.

2. Procédé selon la revendication 1, **caractérisé en ce que** l'intérieur du paquet (1) est alimenté, progressivement par le biais de l'extrémité ouverte de chaque paquet, d'un côté avec un flux d'air chaud stérile, d'un autre côté avec un flux d'agent de stérilisation gazeux et également avec un flux d'air stérile.

3. Procédé selon la revendication 1, **caractérisé en ce qu'**un agent de stérilisation gazeux est ajouté en mélange progressivement dans le flux d'air chaud fourni initialement dans la direction de la machine (MD).

4. Procédé selon la revendication 1, **caractérisé en ce que** la définition radiale du flux vers l'intérieur dans le paquet (1) est limitée par un flux de retour dans la région centrale de chaque paquet.

5. Procédé selon l'une quelconque des revendications précédentes, **caractérisé en ce que** le flux est fourni au moyen d'au moins deux canaux (2) par extrémité de paquet ouverte.

6. Procédé selon la revendication 4, **caractérisé en ce que** les canaux (2) sont inclinés de telle sorte que les directions de flux de ces derniers tombent à l'extérieur de l'un et de l'autre des plans de symétrie qui ont l'axe de symétrie (H) qui coupe, à angle droit, l'ouverture du paquet sous la forme d'une ligne commune, à savoir sont inclinés dans une première direction de manière quelque peu périphérique et sont inclinés dans une seconde direction légèrement vers le centre du paquet (1).

7. Procédé selon la revendication 6, **caractérisé en ce que** les canaux (2) forment un angle inférieur ou égal à 8 degrés dans chacune des première et seconde directions, respectivement.

8. Procédé selon l'une quelconque des revendications précédentes, **caractérisé en ce que** le flux provenant des buses est conservé de manière continue au fil du temps.

9. Procédé selon l'une quelconque des revendications précédentes, **caractérisé en ce que** le flux de gaz fourni, au moins lorsqu'il se compose exclusivement d'un agent de stérilisation, est recyclé pour être réutilisé puisqu'un orifice de sortie (6) est ménagé de manière centrale au niveau de l'ouverture de chaque paquet respectif (1).

10. Appareil dans une machine de remplissage pour fournir un gaz ou des mélanges de gaz à l'intérieur de paquets (1) avant un remplissage ultérieur et une fermeture étanche de ces derniers (1), **caractérisé en ce qu'**il comprend une buse (3) pour un flux de gaz ou d'un mélange de gaz qui comprend au moins un canal (2) pour un gaz ou un mélange de gaz qui est incliné par rapport à au moins un plan contenant l'axe parmi les axes géométriques principaux (H) du paquet qui coupe l'ouverture du paquet à angle droit de telle sorte que le flux de ce dernier, lorsqu'il atteint la paroi interne du paquet, développe un flux défini à la fois radialement et vers l'intérieur tout en formant un vortex de flux hélicoïdal.

11. Appareil selon la revendication 10, **caractérisé en ce que** les canaux (2) sont inclinés de telle sorte que leur direction de flux tombe à l'extérieur de plans de symétrie qui sont parallèles à l'un quelconque des plans de symétrie qui ont l'axe de symétrie (H) qui coupe l'ouverture du paquet (1) à angle droit sous la forme d'une ligne commune, et sont dirigés dans une première direction de manière quelque peu périphérique et dans une seconde direction légèrement vers le centre du paquet (1) .

12. Appareil selon la revendication 11, **caractérisé en ce que** l'angle des canaux (2) est inférieur ou égal à 8 degrés dans chaque direction respective.

13. Appareil selon l'une quelconque des revendications 10 à 12, **caractérisé en ce qu'**il comprend un équipement de recyclage des gaz sous la forme d'un orifice de sortie (6) qui est placé au centre en association avec l'ouverture de chaque paquet respectif (1).
